# EUROPEAN PATENT APPLICATION

(11) **EP 4 706 522 A1**
(43) Date of publication of application: **11.03.2026**
(21) Application number: 24939217.6
(22) Date of filing: 24.05.2024
(51) Int. Cl.: A61B 5/0245, A61B 5/296, A61B 5/313, A61B 5/389, A61B 5/256, A61B 5/25, A61B 5/24, A61B 5/27, A61B 5/00

(54) **WEARABLE DEVICE**

(71) Applicant: Ascend Technology Limited, Hong Kong 999077 (HK)
(72) Inventor: QI, Xin, Shenzhen, Guangdong 518108 (CN); ZHOU, Xin, Shenzhen, Guangdong 518108 (CN); LIAO, Fengyun, Shenzhen, Guangdong 518108 (CN)
(74) Representative: Wang, Bo
(86) International application number: PCT/CN2024/095260
(87) International publication number: WO 2025/241188

(57) **Abstract**

A wearable device is provided. The wearable device includes a garment, including an inner surface configured to contact the skin of a user and an outer surface opposite to the skin, wherein: the garment includes a conductive electrode configured to electrically connect a first region of the inner surface and a second region of the outer surface, the first region of the inner surface is configured to receive an electrical signal generated by the body of the user, and the second region of the outer surface is configured to connect to an external electrode such that the electrical signal is transmitted to the external electrode via the conductive electrode.

## Description

### TECHNICAL FIELD

The present disclosure relates to the field of electronic device, and in particular to a wearable device.

### BACKGROUND

As people pay increasing attention to exercise and health, heart rate during the exercise has gradually become an essential monitoring indicator of people. Exercisers can better grasp their health status by understanding changes in their heart rate, and adjust exercise intensity and guide training plans based on the heart rate, so as to gradually improve exercise performance.

A heart rate strap is commonly used to monitor the heart rate during exercise. The heart rate strap needs to be in close contact with the skin during use, so that two electrocardiogram electrodes on the heart rate strap can acquire a potential difference between potentials at positions of the two electrocardiogram electrodes to obtain an electrocardiogram signal. Since the electrocardiogram electrodes need to be in close contact with the skin, the heart rate strap requires removing or lifting other garment when wearing, which imposes a high requirement for privacy in wearing scenarios. Meanwhile, to ensure close contact between the electrocardiogram electrodes and the skin, the heart rate strap is often made by warp knitting or weft knitting highly elastic base materials, which results in low wearing comfort of the heart rate strap obtained based on this. In addition, since the contact between the heart rate strap and the garment generates static electricity, the static electricity affects electrical potential values acquired by the two electrocardiogram electrodes, thereby causing an interference to the acquired electrocardiogram signal.

Therefore, it is necessary to provide a wearable device, which can expand wearing scenarios of exercise heart rate monitoring devices, improve the wearing comfort of the user, and avoid the static electricity generated by the heart rate strap and the garment to ensure the accuracy of the acquired electrocardiogram signal.

### SUMMARY

One or more embodiments of the present disclosure provide a wearable device. The wearable device includes: a garment, including an inner surface configured to contact the skin of a user and an outer surface opposite to the skin, wherein: the garment includes a conductive electrode configured to electrically connect a first region of the inner surface and a second region of the outer surface, the first region of the inner surface is configured to receive an electrical signal generated by the body of the user, and the second region of the outer surface is configured to connect to an external electrode such that the electrical signal is transmitted to the external electrode via the conductive electrode.

In some embodiments, the first region includes two first conductive regions, the two first conductive regions are disposed on a chest portion of the garment or are disposed on a waist portion of the garment; and when the user wears the garment, the two first conductive regions are located on opposite sides of a midsagittal plane of the body of the user.

In some embodiments, the second region includes two second conductive regions, one of the two second conductive regions is electrically connected to one of the two first conductive regions, and the other of the two second conductive regions is electrically connected to the other of the two first conductive regions; and for each second conductive region, a position of the second conductive region and a position of the first conductive region electrically connected to the second conductive region at least partially overlap along a circumferential direction and an axial direction of the body of the user.

In some embodiments, the second region includes two second conductive regions, one of the two second conductive regions is electrically connected to one of the two first conductive regions, and the other of the two second conductive regions is electrically connected to the other of the two first conductive regions; and for each second conductive region, a position of the second conductive region and a position of the first conductive region electrically connected to the second conductive region do not overlap along a circumferential direction and an axial direction of the body of the user.

In some embodiments, the conductive electrode includes one or more conductive threads, a first portion of the one or more conductive threads is located on the outer surface, and a second portion of the one or more conductive threads extends to the inner surface and is electrically connected to the first portion of the one or more conductive threads.

In some embodiments, the first region and the second region are at least woven by one or more insulating threads, and the one or more conductive threads are interwoven through the inner surface and the outer surface.

In some embodiments, the second portion of the one or more conductive threads is woven to form a corrugated structure in the first region.

In some embodiments, the first region and the second region are formed by interweaving one or more insulating threads and the one or more conductive threads.

In some embodiments, each of the one or more conductive threads is at least made by twisting a conductive fiber around an elastic fiber; or the each of the one or more conductive threads is made by coating an outer side of the conductive fiber with an elastic film.

In some embodiments, the conductive electrode includes at least one set of conductive patches; and for each set of conductive patches, one conductive patch of the set is disposed on the inner surface, another conductive patch of the set is disposed on the outer surface, and the conductive patch disposed on the inner surface and the conductive patch disposed on the outer surface are electrically connected by at least partially contacting each other.

In some embodiments, each conductive patch includes a conductive film or a metal electrode.

In some embodiments, each conductive patch includes a plurality of sub-conductive electrodes and wires electrically connecting the plurality of sub-conductive electrodes.

In some embodiments, the garment further includes an anti-slip region; and the anti-slip region is located within the first region, or the anti-slip region is a peripheral region surrounding the first region.

In some embodiments, the anti-slip region is formed by weaving one or more anti-slip threads; or the anti-slip region is coated with an anti-slip material.

In some embodiments, waterproof structures are respectively disposed around the first region and the second region.

In some embodiments, the first region is connected to other regions of the garment via at least one of the waterproof structures.

In some embodiments, at least one of the waterproof structures is formed by weaving a waterproof fiber; or the at least one of the waterproof structures is formed by permeating a waterproof agent around the first region.

In some embodiments, the garment further includes a first waterproof layer and a second waterproof layer; and the first region is disposed on a side of the first waterproof layer facing the skin, and the second region is disposed on a side of the second waterproof layer facing away from the skin.

In some embodiments, the second region includes two second conductive regions, and a corrugated structure is arranged between the two second conductive regions.

### BRIEF DESCRIPTION OF THE DRAWINGS

The present disclosure is further described by way of exemplary embodiments. The exemplary embodiments are described in detail with reference to the accompanying drawings. These embodiments are not limiting. In these embodiments, the same numerals refer to the same structures, wherein:
FIG. 1 is a schematic diagram illustrating an application scenario of a wearable device according to some embodiments of the present disclosure.
FIG. 2 is a block diagram illustrating an exemplary structure of a wearable device according to some embodiments of the present disclosure.
FIG. 3A is a schematic diagram illustrating an inner surface of a garment according to some embodiments of the present disclosure.
FIG. 3B is another schematic diagram illustrating an inner surface of a garment according to some embodiments of the present disclosure.
FIG. 4A is a schematic diagram illustrating an outer surface of a garment according to some embodiments of the present disclosure.
FIG. 4B is another schematic diagram illustrating an outer surface of a garment according to some embodiments of the present disclosure.
FIG. 5 is a schematic diagram illustrating a human body structure according to some embodiments of the present disclosure.
FIG. 6 is a schematic diagram illustrating a configuration manner of a conductive thread according to some embodiments of the present disclosure.
FIG. 7 is a schematic diagram illustrating a weaving thread mixed with a conductive thread according to some embodiments of the present disclosure.
FIG. 8 is a schematic diagram illustrating a conductive patch according to some embodiments of the present disclosure.
FIG. 9 is another schematic diagram illustrating a conductive patch according to some embodiments of the present disclosure.
FIG. 10 is a schematic diagram illustrating a waterproof configuration of a garment according to some embodiments of the present disclosure.
FIG. 11 is another schematic diagram illustrating a waterproof configuration of a garment according to some embodiments of the present disclosure.

### DETAILED DESCRIPTION

To more clearly illustrate the technical solutions in the embodiments of the present disclosure, the drawings used in the description of the embodiments will be briefly introduced below. Obviously, the drawings in the following description are merely some examples or embodiments of the present disclosure. For a person ordinary skilled in the art, the present disclosure may be applied to other similar scenarios based on these drawings without creative efforts. Unless obviously obtained from the context or the context illustrates otherwise, the same numeral in the drawings refers to the same structure or operation.

It should be understood that the terms "system," "device," "unit," and/or "module" used herein are a manner for distinguishing components, elements, parts, sections, or assemblies of different levels. However, if other words can achieve the same purpose, the words may be replaced by other expressions.

As shown in the present disclosure and the claims, unless the context clearly indicates an exception, the words "a," "an," "one," and/or "the" are not specifically limited to the singular form, and may also include the plural form. Generally, the terms "include" and "comprise" only indicate that clearly identified steps and elements are included, and these steps and elements do not constitute an exclusive list. A method or device may also include other steps or elements.

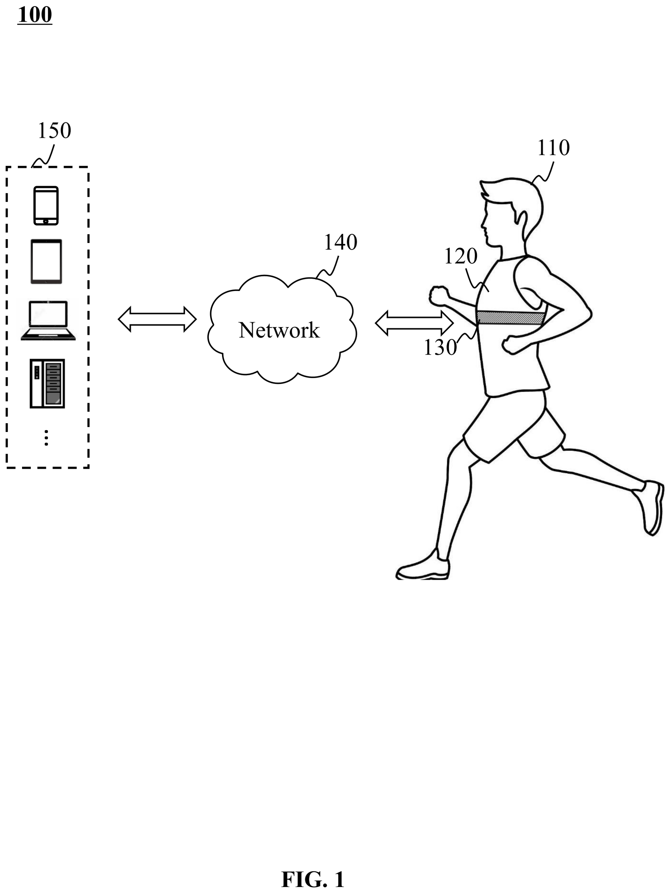

FIG. 1 is a schematic diagram illustrating an application scenario of a wearable device according to some embodiments of the present disclosure.

An application scenario 100 of a wearable device (hereinafter referred to as the application scenario 100) may be used to monitor an electrical signal generated by the body of a user. The electrical signal generated by the body of the user may include an electrocardiogram signal of a user 110. The electrical signal generated by the body of the user may also include other content. For example, the electrical signal may also include an electromyographic signal of the user 110.

As shown in FIG. 1, the application scenario 100 of the wearable device includes the user 110, a monitoring device 130, a wearable device 120, a network 140, and a terminal device 150. The user may wear the wearable device 120. The wearable device 120 may be provided with the monitoring device 130 electrically connected to the wearable device 120. The monitoring device 130 may receive the electrical signal generated by the body of the user through the wearable device 120 and perform monitoring, and transmit the electrical signal to the terminal device 150 through the network 140.

The user 110 refers to an object that requires monitoring of the electrical signal generated by the body.

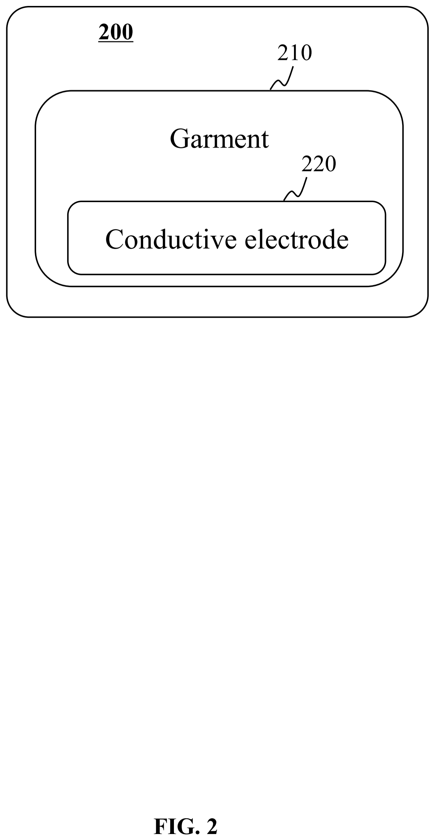

The wearable device 120 is configured to assist a device for monitoring the electrical signal generated by the body of the user. As shown in FIG. 1, the user 110 may wear the wearable device 120. The wearable device 120 may transmit the electrical signal (not shown in FIG. 1) generated by the body of the user to the monitoring device 130, so that the monitoring device 130 performs monitoring. In some embodiments, the wearable device 120 may include a garment having a conductive electrode. When the user 110 wears the garment, the conductive electrode is electrically connected to the monitoring device 130. The conductive electrode may transmit the electrical signal received by an inner surface of the garment to the monitoring device 130, so that the monitoring device 130 performs monitoring. More descriptions regarding the above embodiments may be found in related descriptions below in the present disclosure, e.g., FIG. 2 and related descriptions thereof.

The monitoring device 130 refers to a device configured to monitor the electrical signal generated by the body of the user. It is understood that the monitoring device 130 may be different when monitoring different electrical signals. For example, when an electrocardiogram signal generated by the body of the user needs to be monitored, the monitoring device 130 may be a heart rate strap. As another example, when an electromyographic signal generated by the body of the user needs to be monitored, the monitoring device 130 may be an electromyographic signal monitoring strap. As shown in FIG. 1, the wearable device 120 may be provided with one monitoring device 130 or a plurality of same or different monitoring devices 130.

As shown in FIG. 1, the monitoring device 130 may be provided on an outer surface of the wearable device 120, so as to receive the electrical signal generated by the body of the user and transmitted by the wearable device 120. A plurality of electrodes may be provided on the monitoring device 130. The plurality of electrodes may be connected to the conductive electrode of the wearable device 120 to acquire the electrical signal generated by the body of the user. For example, when the monitoring device 130 is the heart rate strap, two electrodes may be provided on the heart rate strap. The two electrodes are connected to two different regions of the wearable device 120 to obtain electrical potentials at two different positions of the body of the user. The electrocardiogram signal of the user 110 may be obtained based on an electrical potential difference between the two different positions.

The network 140 may connect various components of the application scenario 100 (e.g., the terminal device 150, the monitoring device 130, etc.) and/or connect the application scenario 100 to an external resource portion. The network 140 enables communication among the various components of the application scenario 100 and other external portions beyond the application scenario 100, facilitating exchange of data and/or information. For example, the terminal device 150 may obtain the electrical signal generated by the body of the user and acquired by the monitoring device 130 through the network 140.

The network 140 may be any form of a wired or wireless network, or any combination thereof. Merely by way of example, the network 140 may include a cable network, a wired network, a fiber optic network, a telecommunication network, an intranet, the Internet, a local region network (LAN), a wide region network (WAN), a wireless local region network (WLAN), a metropolitan region network (MAN), a public switched telephone network (PSTN), a Bluetooth network, a ZigBee network, a near field communication (NFC) network, etc., or any combination thereof. The network 140 may include at least one network access point, and at least one component of the application scenario 100 may be connected to the network 140 through the at least one network access point to exchange data and/or information. For example, the acquired electrical signal generated by the body of the user may be transmitted through the network 140.

The terminal device 150 refers to a device and/or software used by a user related to the application scenario 100. The user related to the application scenario 100 includes, but is not limited to, the user 110, a physician (e.g., a clinician or a radiotherapist), a nurse, etc. For example, the terminal device 150 may be a device or software configured to control the monitoring device 130. The user may issue a control instruction to the monitoring device 130 through the terminal device 150, thereby controlling the monitoring device 130 to acquire the electrical signal generated by the body of the user. For example, the terminal device 150 may send the control instruction input by the user to the monitoring device 130 through the network 140, to control the monitoring device 130 to acquire the electrical signal generated by the body of the user 110. In some embodiments, the terminal device 150 may obtain the electrical signal generated by the body of the user 110 and acquired by the monitoring device 130 through the network 140. In some embodiments, the terminal device 150 may be a mobile device, a tablet computer, a laptop computer, a desktop computer, or other devices having input and/or output functions, or any combination thereof.

It should be noted that the above descriptions of the application scenario 100 is merely for example and illustration, and does not limit the applicable scope of the present disclosure. For a person skilled in the art, various modifications and changes may be made to the application scenario 100 under the guidance of the present disclosure. However, these modifications and changes are still within the scope of the present disclosure. For example, the application scenario 100 may further include a storage device (not shown in FIG. 1). The storage device may store data, instructions, and/or any other information (e.g., the storage device may store data obtained from the monitoring device 130 and/or the terminal device 150). As another example, various components in the application scenario 100 may be integrated together or independently provided (e.g., the storage device may be provided in the terminal device 150).

FIG. 2 is a block diagram illustrating an exemplary structure of a wearable device according to some embodiments of the present disclosure.

A wearable device 200 may assist a monitoring device (e.g., the monitoring device 130 shown in FIG. 1) in monitoring an electrical signal generated by a body of a user.

As shown in FIG. 2, in some embodiments, the wearable device 200 includes a garment 210 having a conductive electrode 220. The garment 210 includes an inner surface configured to contact the skin of the user and an outer surface opposite to the skin. The conductive electrode 220 is provided on the garment 210. The conductive electrode 220 is configured to transmit an electrical signal generated by the body of the user from the inner surface to the outer surface of the garment 210.

During use, the user first wears the wearable device 200, then attaches the monitoring device to an outer surface of the wearable device 200, and acquires the electrical signal transmitted to the outer surface through the monitoring device.

The garment 210 refers to various clothes capable of receiving the electrical signal (e.g., an electrocardiogram signal) generated by the body of the user. For example, the garment 210 may be a T-shirt, a sports bra, etc. In some embodiments, the garment 210 is made of a soft, form-fitting, and highly elastic fabric, which ensures that the garment 210 closely contacts the skin of the user, thereby ensuring the monitoring accuracy of the monitoring device. Merely by way of example, the fabric of the garment 210 may include a plant fiber (e.g., a cotton fiber, a hemp fiber, etc.), an animal fiber (e.g., wool, etc.), a synthetic fiber (e.g., an acrylic fiber, a polyester fiber, etc.), etc. In some embodiments, the garment 210 may generate a corresponding deformation based on a change in a movement of the user. For example, when the user breathes, the garment 210 generates a deformation with inhalation and exhalation actions of the user, which ensures that the conductive electrode 220 disposed in the garment 210 maintains close contact with the skin of the user.

In some embodiments, a first region 211 is provided on the inner surface, a second region 212 is provided on the outer surface. The garment 210 may receive the electrical signal generated by the body of the user through the first region 211. The first region 211 and the second region 212 are electrically connected via the conductive electrode 220. The second region 212 may transmit the electrical signal generated by the body of the user to an external electrode. More descriptions regarding the first region 211, the second region 212, the conductive electrode 220, and the external electrode may be found in the related descriptions below in the present disclosure.

In some embodiments, the garment 210 further includes an anti-slip region. The anti-slip region is configured to increase a friction force between a corresponding region and the skin, preventing relative sliding between the corresponding region and the skin.

The anti-slip region may be in any shape. For example, a shape of the anti-slip region includes, but is not limited to, one or more of a square, a circle, a circular ring, a triangle, a corrugated shape, etc. The anti-slip region may be a single continuous region or include a plurality of relatively independent sub-anti-slip regions, and shapes of the plurality of relatively independent sub-anti-slip regions may be the same or different.

The anti-slip region is disposed on the inner surface of the garment 210 to prevent relative sliding between the first region 211 and the skin. In some embodiments, the anti-slip region is located within the first region 211. When the anti-slip region is located within the first region 211, a shape and an area of the anti-slip region may be the same as or different from those of the first region 211. In some embodiments, the anti-slip region is a peripheral region surrounding the first region 211. For example, the anti-slip region is disposed around an edge of the first region 211. The anti-slip region may also be disposed on the inner surface of the garment 210 in other manners. For example, the anti-slip region may be partially located within the first region 211 and partially located outside the first region 211.

In some embodiments, the anti-slip region is implemented in a plurality of manners to achieve an anti-slip effect thereof. In some embodiments, the anti-slip region is formed by weaving one or more anti-slip threads. Each of the one or more anti-slip threads is made of one or more anti-slip materials. For example, the each of one or more anti-slip threads is made of one or more of silicone, polyvinyl chloride, etc. In some embodiments, the anti-slip region is coated with an anti-slip material. For example, when the anti-slip region is located within the first region 211, the anti-slip material is coated on a portion of the first region 211 to form the anti-slip region. The anti-slip material includes, but is not limited to, silicone, rubber, polyvinyl chloride, etc.

In some embodiments of the present disclosure, the friction force between the first region 211 and the skin is increased by providing the anti-slip region, to prevent the relative sliding between the first region 211 and the skin, which can increase the stability when the first region 211 receives the electrical signal generated by the body of the user, and avoid fluctuations in the acquired electrical signal generated by the body of the user due to the relative sliding.

The conductive electrode 220 is configured to electrically connect the first region 211 of the inner surface and the second region 212 of the outer surface. In some embodiments, the first region 211 of the inner surface may be a region where the conductive electrode 220 contacts the skin of the user, i.e., the region where the conductive electrode 220 contacts the skin of the user constitutes a portion of the inner surface of the garment 210. The second region 212 of the outer surface may be a region where the conductive electrode 220 is opposite to the skin, i.e., the region where the conductive electrode 220 is opposite to the skin constitutes a portion of the outer surface of the garment 210. The electrical signal generated by the body of the user is transmitted to the second region 212 of the outer surface via the conductive electrode 220 after passing through the first region 211 of the inner surface, and is transmitted to the external electrode via the second region 212. The external electrode refers to an electrode of the monitoring device. For example, when the monitoring device is a heart rate strap, the external electrode is an electrode of the heart rate strap.

The first region 211 is configured to receive the electrical signal generated by the body of the user. It is understood that the first region 211 needs to be disposed on a corresponding region of the body when acquiring the electrical signal generated by the body of the user, to ensure the accuracy of the first region 211 receiving the electrical signal. For example, when acquiring the electrocardiogram signal, the first region 211 needs to be disposed on a region of the body where the electrocardiogram signal can be acquired (e.g., a chest portion or a waist portion).

In some embodiments, the first region 211 includes two first conductive regions 2111. When the user wears the garment 210, the two first conductive regions 2111 are located on opposite sides of a midsagittal plane of the body of the user. Sizes and shapes of the two first conductive regions 2111 may be the same or different.

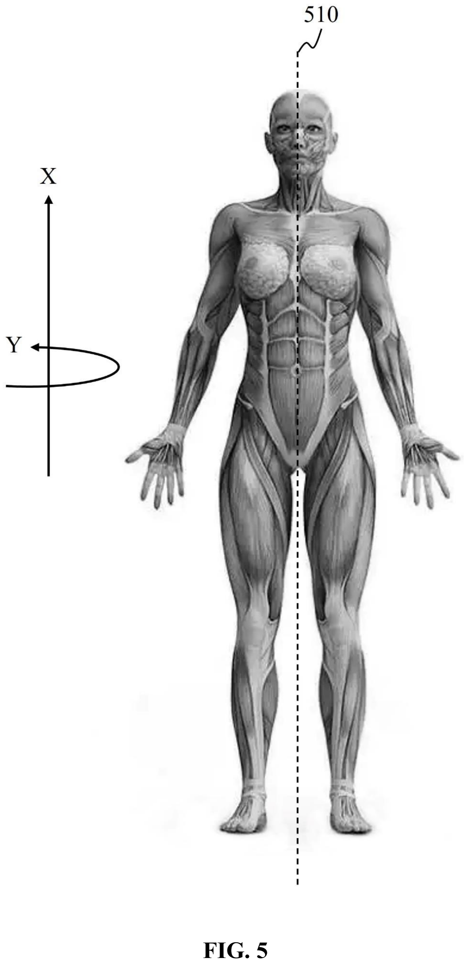

As shown in FIG. 5, the midsagittal plane of the human body refers to a plane passing through a midline 510 of the human body and dividing the human body into two equal or approximately equal parts. The midline 510 of the human body is determined based on a line from a tip of the nose of the human body to a midpoint between two nipples, a line from the midpoint between the two nipples to a midpoint of the abdominal navel, or a line from the midpoint of the abdominal navel to a midpoint of the pubic symphysis joint.

In some embodiments, the two first conductive regions 2111 are symmetrically disposed relative to the midsagittal plane. In some embodiments, the two first conductive regions 2111 are not symmetrically disposed relative to the midsagittal plane. When the two first conductive regions 2111 are not symmetrically disposed relative to the midsagittal plane, a distance difference between each of the two first conductive regions 2111 and the midsagittal plane is less than a preset distance threshold. For example, a distance threshold is in range of 100 mm to 300 mm. The preset distance threshold is selected from the aforementioned range based on user differences. For example, the different preset distance threshold is determined based on a size of the garment 210 (e.g., a clothing size). When the size of the garment 210 is large, the determined preset distance threshold is large; and when the size of the garment 210 is small, the determined preset distance threshold is small, which ensures monitoring effect for users with different body types. By restricting the positions of the two first conductive regions 2111 relative to the midsagittal plane, motion artifacts of the two conductive electrodes 220 exhibit good consistency, which facilitates eliminating the motion artifacts. For example, a differential amplification circuit is configured to eliminate the motion artifacts in the electrocardiogram signal, thereby improving the quality of the electrocardiogram signal.

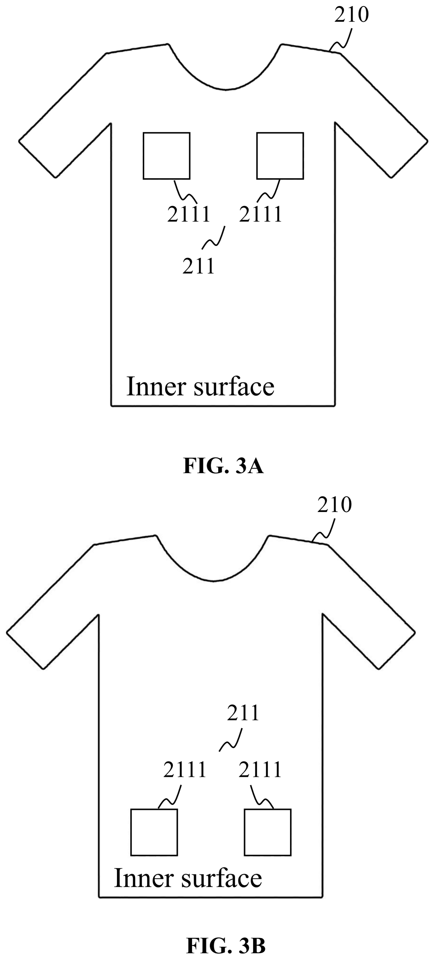

In some embodiments, the two first conductive regions 2111 may be disposed on a chest portion of the garment 210. The midsagittal plane of the human body may divide the chest portion of the human body into a left chest region and a right chest region. As shown in FIG. 3A, one first conductive region 2111 may be disposed on the left chest region of the garment 210, and the other first conductive region 2111 may be disposed on the right chest region of the garment 210.

In some embodiments, the two first conductive regions 2111may be disposed on a waist portion of the garment 210. The midsagittal plane of the human body may divide the waist portion of the human body into a left waist region and a right waist region. As shown in FIG. 3B, one first conductive region 2111 may be disposed on the left waist region of the garment 210, and the other first conductive region 2111 may be disposed on the right waist region of the garment 210. Merely by way of example, when the user wears the garment 210, the two first conductive regions 2111 respectively contact an ilium on each side of a trunk centerline and a region adjacent to the ilium.

The second region 212 is configured to connect to the external electrode.

In some embodiments, the second region 212 includes two second conductive regions 2121, and sizes and shapes of the two second conductive regions 2121 may be the same or different. One of the two second conductive regions 2121 is electrically connected to one of the two first conductive regions 2111, and the other of the two second conductive regions 2121 is electrically connected to the other of the two first conductive regions 2111. Each second conductive region 2121 may receive the electrical signal generated by the body of the user through the first conductive region 2111 electrically connected to the second conductive region 2121.

A position of each second conductive region 2121 needs to correspond to a position of an electrode in a monitoring device after the user wears the monitoring device, so that the electrical signal generated by the body of the user is transmitted to the monitoring device through the second conductive region 2121. The shape and size of each second conductive region 2121 may be the same as or different from a shape of the electrode in the monitoring device. For example, the shape of each second conductive region 2121 may be the same as the shape of the electrode in the monitoring device, and an area of each second conductive region 2121 may be larger than an area of the electrode in the monitoring device.

When the monitoring device is the heart rate strap, after the user wears the wearable device 200 and the heart rate strap, one of two electrodes on the heart rate strap is electrically connected to one of the two second conductive regions 2121, and the other electrode on the heart rate strap is electrically connected to the other second conductive region 2121, so that the electrical signal generated by the body of the user is acquired through the two second conductive regions 2121.

In some embodiments, for each second conductive region 2121, the second conductive region 2121 and the first conductive region 2111 electrically connected to the second conductive region 2121 at least partially overlap along a circumferential direction and an axial direction of the body of the user. The axial direction of the body of the user is a direction perpendicular to the ground when the human body stands, for example, a direction indicated by an arrow X in FIG. 5. The circumferential direction of the body of the user is a direction around the human body when the human body stands, for example, a direction indicated by an arrow Y in FIG. 5.

In some embodiments, the second conductive region 2121 and the first conductive region 2111 electrically connected to the second conductive region 2121 completely overlap along the circumferential direction and the axial direction of the body of the user. When the two first conductive regions 2111 are both disposed on the chest portion of the garment 210 or both disposed on the waist portion of the garment 210, sizes and positions of the two second conductive regions 2121 disposed on the outer surface of the garment 210 may completely correspond to sizes and positions of the two first conductive regions 2111, respectively.

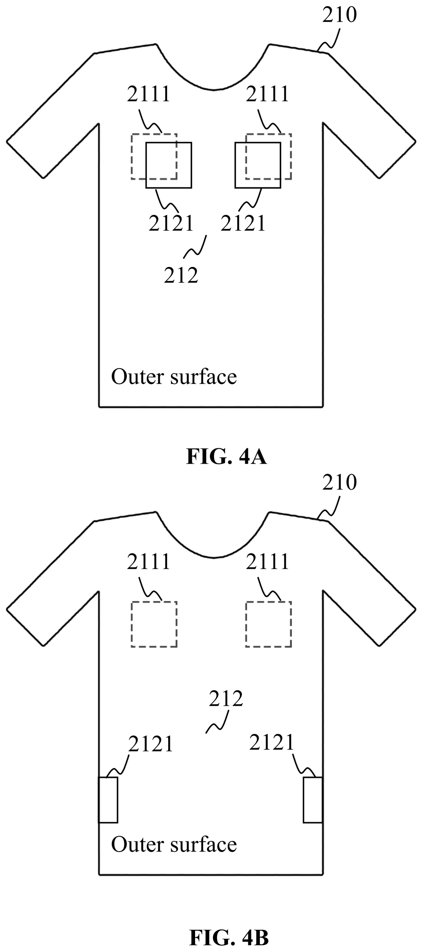

In some embodiments, the second conductive region 2121 and the first conductive region 2111 electrically connected to the second conductive region 2121 partially overlap along the circumferential direction and the axial direction of the body of the user. For example, FIG. 4A shows an outer surface of the garment 210. For each second conductive region 2121, the second conductive region 2121 and the first conductive region 2111 electrically connected to the second conductive region 2121 partially overlap along the circumferential direction and the axial direction of the body of the user. It is worth noting that, in order to illustrate a positional relationship between the second conductive region 2121 and the first conductive region 2111, the first conductive region 2111 is represented by a dashed line on the outer surface of the garment 210 shown in FIG. 4A and FIG. 4B, while the first conductive region 2111 is actually located on the inner surface of the garment 210.

In some embodiments of the present disclosure, the second conductive region 2121 and the first conductive region 2111 electrically connected to the second conductive region 2121 are set to at least partially overlap along the circumferential direction and the axial direction of the body of the user, which facilitates achieving the electrical connection between the first conductive region 2111 and the second conductive region 2121 corresponding to the first conductive region 2111. At the same time, the configuration can also reduce a total projected area of the first region 211 and the second region 212 on the garment 210. It is understood that a material in a region where the conductive electrode 220 is disposed may have a certain heterogeneity in tactile sensation compared to materials in other regions of the garment 210. Regions with heterogeneity can also be reduced by reducing the total projected area of the first region 211 and the second region 212 on the garment 210, thereby improving the comfort when the user wears the garment. In addition, the configuration can also ensure that the wearable device 200 is compatible with various monitoring devices on the market, which avoids the need for the user to purchase a monitoring device specifically matched with the wearable device 200, thereby reducing a usage cost for the user.

In some embodiments, for each second conductive region 2121, the second conductive region 2121 and the first conductive region 2111 electrically connected to the second conductive region 2121 do not overlap along the circumferential direction and the axial direction of the body of the user. For example, FIG. 4B shows an outer surface of the garment 210. The two second conductive regions 2121 are both disposed on a waist portion of the outer surface, while the two first conductive regions 2111 are both disposed on a chest portion of the outer surface. The two second conductive regions 2121 and the two first conductive regions 2111 do not overlap along the circumferential direction and the axial direction of the body of the user.

In some embodiments of the present disclosure, the second conductive region 2121 and the first conductive region 2111 electrically connected to the second conductive region 2121 are set to not overlap along the circumferential direction and the axial direction of the body of the user, which enables the position selection of the second region 212 to be more flexible on the basis of acquiring the electrical signal generated by the body of the user. It is understood that, as described above, the position of the second conductive region 2121 corresponds to the position of the electrode in the monitoring device. Based on this, the position of the second conductive region 2121 can be set to meet different requirements. For example, the second region 212 may be disposed on a rear side of the waist portion of the user to reduce a foreign body sensation when the user uses the monitoring device, thereby improving the comfort. As another example, the second region 212 may be disposed on the back of the user to avoid contact or collision of the user with the monitoring device during a movement of the user, which may affect accuracy of the acquired electrical signal.

In some embodiments, a friction enhancement region including a concave-convex structure is arranged between the two second conductive regions 2121. For example, a weaving pattern of a fabric between the two second conductive regions 2121 of the garment 210 is a corrugated structure. The corrugated structure can increase a contact area between the monitoring device and a corresponding region in various directions. Regardless of a direction of a force, the corrugated structure can provide a good grip force, thereby increasing a friction force between the garment 210 and the monitoring device to reduce relative sliding between the garment 210 and the monitoring device, so as to improve stability when the monitoring device acquire the electrical signal. In some alternative embodiments, the concave-convex structure may be a protruding dot array made of an anti-slip material or other patterns or designs.

In some embodiments, the conductive electrode 220 may be disposed in a plurality of manners to achieve an electrical connection between the first region 211 and the second region 212. For example, the conductive electrode 220 may include one or more conductive threads, and the electrical connection between the first region 211 and the second region 212 is achieved based on the aforementioned one or more conductive threads. As another example, the conductive electrode 220 may further include conductive patches. The conductive patches disposed on the first region 211 and the conductive patches disposed on the second region 212 at least partially contact each other, thereby achieving the electrical connection between the first region 211 and the second region 212. More descriptions regarding the aforementioned configuration manners of the conductive electrode 220 may be found in related descriptions below in the present disclosure.

In some embodiments, configuration manners of the conductive electrode 220 in two sets of first conductive region 2111 and second conductive region 2121 that are electrically connected may be the same or different. For example, electrical connections of the two sets of first conductive region 2111 and second conductive region 2121 are implemented through the one or more conductive threads. As another example, the electrical connection of one set of first conductive region 2111 and second conductive region 2121 is implemented through the one or more conductive threads, and the electrical connection of the other set of first conductive region 2111 and second conductive region 2121 is implemented through the conductive patches.

In some embodiments of the present disclosure, the garment 210 receives the electrical signal generated by the body of the user, and transmits the aforementioned electrical signal from the inner surface of the garment 210 to the outer surface through the conductive electrode 220, so that the electrical signal can be acquired by the electrode of the monitoring device. The configuration can expand wearing scenarios for the monitoring device. For example, the monitoring device can be worn or removed in any scenarios on the basis of wearing the wearable device 200. At the same time, static electricity generated by continuous friction between the monitoring device and the garment during movement can be avoided by wearing the monitoring device on the outer surface of the garment 210, which ensures the accuracy of the acquired electrical signal. In addition, direct contact between the monitoring device and the skin can be avoided, which improves wearing comfort for the user.

Some embodiments below in the present disclosure will describe implementation manners of disposing the conductive electrode through the one or more conductive threads.

In some embodiments, the conductive electrode 220 includes one or more conductive threads 221, a first portion 221-1 of the one or more conductive threads 221 is located on the outer surface, and a second portion 221-2 of the one or more conductive threads 221 extends to the inner surface and is electrically connected to the first portion 221-1 of the one or more conductive threads 221, so as to achieve the electrical connection between the first conductive region 221 and the second conductive region 2121 corresponding to the first conductive region 221. In some embodiments, the first conductive region 2111 may be formed by the second portion 221-2 of the one or more conductive threads 221, and the second conductive region 2121 may be formed by the first portion 221-1 of the one or more conductive threads 221. In some embodiments, the first conductive region 2111 and/or the second conductive region 2121 may be formed by other conductive materials besides the one or more conductive threads 221. The first portion 221-1 and the second portion 221-2 of the one or more conductive threads 221 are electrically connected to the first conductive region 2111 and/or the second conductive region 2121, respectively, to achieve the electrical conduction between the first conductive region 2111 and the second conductive region 2121.

In some embodiments, each of the one or more conductive threads 221 may be configured to have elasticity, thereby ensuring the wearing comfort and integrity of the garment 210.

The one or more conductive threads 221 may be configured to have elasticity in a plurality of manners. In some embodiments, each of the one or more conductive threads is at least made by twisting a conductive fiber around an elastic fiber (e.g., spandex, polyether ester spandex, rubber thread, etc.). The each of the one or more conductive threads may include only the conductive fiber (e.g., a metal fiber, a conductive polymer fiber, etc.) and the elastic fiber. The each of the one or more conductive threads may further include an insulating thread (e.g., a cotton thread) to ensure the wearing comfort of the garment 210. In some embodiments, the each of the one or more conductive threads 221 is made by coating an outer side of the conductive fiber with an elastic film. Merely by way of example, before being coated with the elastic film, a stretchable displacement may be reserved in the conductive fiber (e.g., by placing the conductive fiber in a curved structure). On this basis, coating the conductive fiber with the elastic film may make the finally formed conductive thread extensible. The elastic film may include, but is not limited to, a spandex film, a polyester-spandex film, a rubber film, etc.

The first region 211 and the second region 212 include at least a region formed by weaving one or more insulating threads 213 and/or elastic threads.

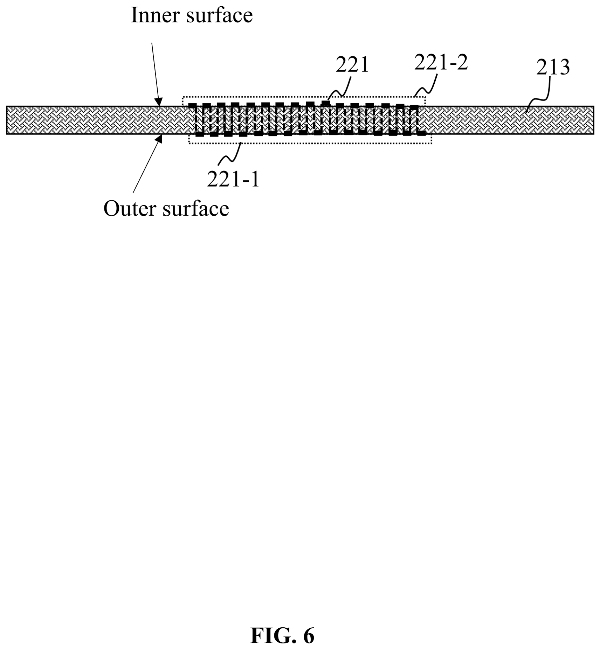

In some embodiments, to make the first region 211 and the second region 212 conductive, the one or more conductive threads 221 may be further interwoven through the region formed by weaving the one or more insulating threads 213 and/or elastic threads, which allows the one or more conductive threads 221 to be interwoven through the inner surface and the outer surface, thereby forming the first portion 221-1 and the second portion 221-2 that are electrically connected. As shown in FIG. 6, the first portion 221-1 of the one or more conductive threads 221 includes a portion of the one or more conductive threads 221 located on the outer surface. The second portion 221-2 of the one or more conductive threads 221 includes a portion of the one or more conductive threads 221 located on the inner surface. The first portion 221-1 and the second portion 221-2 are electrically conducted via the one or more conductive threads 221 through a woven region, thereby achieving an electrical connection between the first region 211 and the second region 212.

To realize the structure of the wearable device 200 described in the foregoing embodiments, a weaving process of the wearable device 200 may be: first at least forming the garment 210 by weaving the one or more insulating threads 213, and then interweaving each of the one or more conductive threads 221 through the inner surface and the outer surface of the garment 210, thereby obtaining the wearable device 200.

In some embodiments, the second portion 221-2 of the one or more conductive threads 221 is woven to form a corrugated structure in the first region 211. The corrugated structure can increase a contact area between the first region 211 and the skin in various directions. Regardless of a direction of a force, the corrugated structure can provide a good grip force, thereby increasing a friction force between the first region 211 and the skin to reduce relative sliding between the first region 211 and the skin, so as to improve the stability when the monitoring device acquire the electrical signal.

In some embodiments of the present disclosure, the conductive electrode 220 is configured as the one or more conductive threads 221 interwoven through the inner surface and the outer surface, which allows reprocessing of various different garments, to form the electrical connection between the inner surface and outer surface of each garment. The wearable device 200 produced in this manner has advantages of low cost, simple process, and high adaptability.

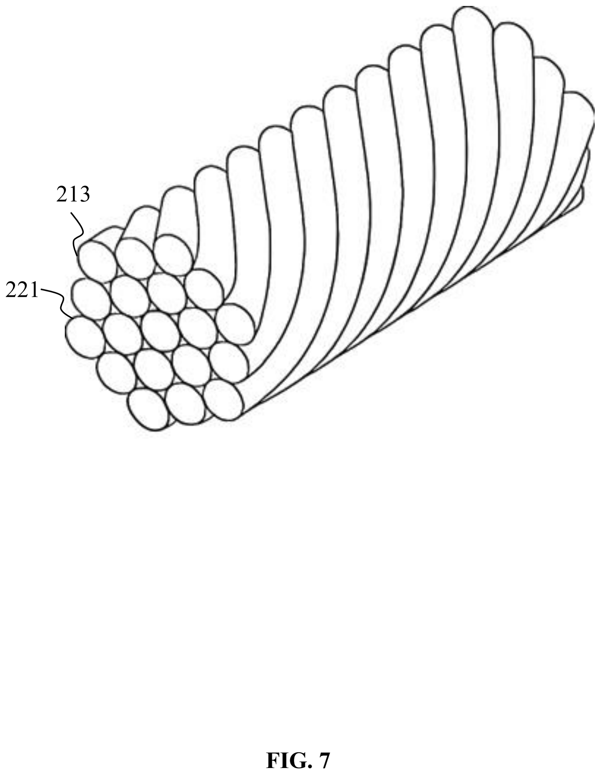

In some embodiments, the first region 211 and the second region 212 are formed by interweaving the one or more insulating threads 213 and the one or more conductive threads 221. The interweaving may include two manners: hybrid yarn formation and in-weave blending. The hybrid yarn formation refers to: mixing a base material (e.g., the insulating threads) and a special material (e.g., the conductive threads) to form one or more integrated weaving threads. Merely by way of example, FIG. 7 shows weaving the garment 210 with a weaving thread formed by interweaving 4 conductive threads 221 and 12 insulating threads 213. One or more ordinary weaving threads (e.g., weaving threads including only the insulating threads) are woven in non-specified regions (e.g., outside the first region 211 and the second region 212), while the aforementioned integrated weaving threads are selected to be woven in specified regions (e.g., the first region 211 and the second region 212), to enable a corresponding region to obtain a specific property (e.g., conductivity). The garment 210 is obtained after completion of weaving. Weaving with pre-mixed weaving threads can simplify subsequent weaving operations and reduce production difficulty. Pre-treating the weaving threads including the special material makes it easier to control a proportion of the special material, which ensures production quality. The in-weave blending refers to: weaving the garment 210 with the ordinary weaving threads, when weaving the specified regions, adding the special material for mixed weaving on the basis of the ordinary weaving threads, or replacing the ordinary weaving threads with the special material, and obtaining the garment 210 after completion of weaving. When weaving the specified regions, since the special material is added, a count of the ordinary weaving threads can be correspondingly reduced to maintain consistency with a count of threads in other regions of the garment 210, which ensures the integrity of the garment 210. Merely by way of example, when weaving the garment 210 with 4 ordinary weaving threads, two conductive threads 221 need to be added when weaving the first region 211the a count of the ordinary weaving threads can be reduced to 2 to ensure that a total count of weaving threads is 4 when weaving the first region 211, thereby maintaining the consistency with other regions of the garment 210. The in-weave blending can improve flexibility during production, allowing adjustment of the proportion of the special material according to different demands at any time. Since no pre-treatment of the weaving threads is required, extra resource consumption from pre-treatment is reduced, which ensures that weaving result is obtained more quickly.

To realize the structure of the wearable device 200 described in the foregoing embodiments, the interweaving process of the wearable device 200 may be: when the loom generates the regions of the garment 210 outside the first region 211 and the second region 212, selecting one or more weaving threads made of the insulating threads 213 for weaving; and when the loom generates the first region 211 and the second region 212, adding one or more weaving threads obtained by interweaving the one or more conductive threads 221 and the one or more insulating threads 213, and obtaining the wearable device 200 after completion of weaving the entire garment 210. The interweaving process of the wearable device 200 may also be: when the loom generates the regions of the garment 210 outside the first region 211 and the second region 212, selecting one or more weaving threads made of the insulating threads 213 for weaving; and when the loom generates the first region 211 and the second region 212, adding one or more weaving threads made of the one or more conductive threads 221 for weaving on the basis of the one or more weaving threads made of the one or more insulating threads 213, and correspondingly reducing a count of the one or more weaving threads made of the one or more insulating threads 213, or directly replacing the one or more insulating threads 213 with the one or more conductive threads 221 for weaving. The wearable device 200 is obtained after completion of weaving the entire garment 210.

In some embodiments of the present disclosure, the first region 211 and the second region 212 are woven with the weaving threads formed by interweaving the one or more conductive threads 221 and the one or more insulating threads 213, which can improve the integrity of the wearable device 200 and improve the wearing comfort.

In some embodiments of the present disclosure, the one or more conductive threads 221 are used to achieve the electrical connection between the first region 211 and the second region 212, which can ensure the integrity of the wearable device 200 and improve wearing comfort.

Some embodiments below in the present disclosure will describe implementation manners of disposing the conductive electrode via the conductive patches.

In some embodiments, the conductive electrode 220 includes at least one set of conductive patches 222. For each set of conductive patches 222, one conductive patch 222 of the set is disposed on the inner surface, another conductive patch 222 of the set is disposed on the outer surface, and the conductive patch 222 disposed on the inner surface and the conductive patch 222 disposed on the outer surface are electrically connected by at least partially contacting each other. For example, for each set of conductive patches 222, the conductive patch 222 disposed on the inner surface and the conductive patch 222 disposed on the outer surface of the each set of conductive patches 222 may partially contact each other, thereby achieving the electrical connection. As another example, for each set of conductive patches 222, the conductive patch 222 disposed on the inner surface and the conductive patch 222 disposed on the outer surface of the each set of conductive patches 222 may completely contact each other, thereby achieving the electrical connection.

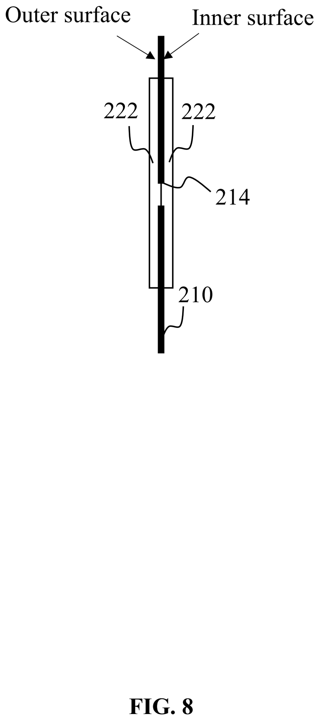

As shown in FIG. 8, a through hole 214 may be provided between the first region 211 and the second region 212 of the garment 210. One conductive patch 222 may contact the inner surface, and another conductive patch 222 may contact the outer surface. The two conductive patches contact at a position where the through hole 214 is located to achieve the electrical connection. A size of the through hole 214 affects a contacting degree between the two conductive patches 222.

Each conductive patch 222 may be of any shape. For example, the conductive patch 222 may be circular, rectangular, triangular, or other shapes. The conductive patch 222 may be disposed on the inner surface or the outer surface in a plurality of manners (e.g., bonding or sewing).

In some embodiments, each conductive patch 222 includes a conductive film or a metal electrode. The two conductive patches 222 of each set of conductive patches 222 may contact via a conductive medium (e.g., conductive adhesive, conductive paste, conductive tape), thereby achieving the electrical connection between the two conductive patches 222.

In some embodiments, each conductive patch 222 includes a plurality of sub-conductive electrodes 2221 and wires 2222 electrically connecting the plurality of sub-conductive electrodes 2221.

It can be understood that, since the plurality of sub-conductive electrodes are electrically connected via the wires 2222, when any one of the plurality of sub-conductive electrodes 2221 on a conductive patch 222 receives an electrical signal, the electrical signal may be transmitted to other of the plurality of sub-conductive electrodes 2221 via the wires 2222. When the external electrode is electrically connected to any one or more of the plurality of sub-conductive electrodes 2221, the external electrode may receive the electrical signal generated by the body of the user.

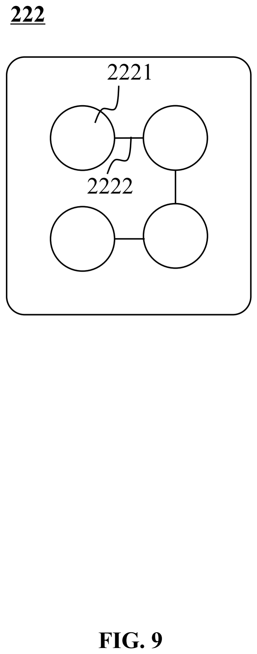

In some embodiments, the plurality of sub-conductive electrodes 2221 may be arranged in an array. As shown in FIG. 9, the conductive patch 222 may include 4 sub-conductive electrodes 2221 arranged in an array. The 4 sub-conductive electrodes 2221 are electrically connected to each other via the wires 2222.

In some embodiments, the wires 2222 connecting the plurality of sub-conductive electrodes 2221 may have an extension tension. Merely by way of example, each wire 2222 may be an elastic wire, and the elastic wire may elastically stretch along an axial direction of the wire 2222. The wire 2222 may be a interweaving wire of a metal wire (e.g., a silver wire or a copper wire) and an elastic thread (e.g., a rubber thread). As another example, the wire 2222 connecting two sub-conductive electrodes 2221 has a certain curvature, and the curvature may allow the wire 2222 to possess certain stretchability. Through the foregoing configuration, when the garment 210 deforms, the wires 2222 also deform correspondingly, which ensures the integrity of the conductive patches 222 with the garment 210, improves softness of a region corresponding to the conductive patches 222, and also increases a friction force between the conductive patches 222 and other portions (e.g., a monitoring device or the skin) to prevent relative sliding.

In some embodiments of the present disclosure, the conductive patches 222 are configured to achieve the electrical connection between the first region 211 and the second region 212. The configuration manner of the conductive electrode 220 has a low production cost, a simple process, and is convenient for processing and production.

It is worth noting that water is conductive. After the user wears the wearable device 200, the first region 211 or the second region 212 may be electrically conducted to other regions of the garment 210 through the water (e.g., rainwater or sweat), which may cause the electrical signal acquired by the monitoring device to have significant noise, affecting detection reliability. In some cases, two first conductive regions (or two second conductive regions) may be electrically conducted, preventing monitoring of the electrocardiogram signal. Based on this, some embodiments of the present disclosure provide a waterproof configuration for the first region 211 and the second region 212 to prevent the water from electrically conducting the first region 211 or the second region 212 to the other regions of the garment 210, thereby ensuring the accuracy of the acquired electrical signal.

In some embodiments, waterproof structures 215 are disposed around the first region 211 and the second region 212 to prevent the water from electrically conducting the first region 211 or the second region 212 to the other regions. In some embodiments, waterproof layers are disposed on the first region 211 and the second region 212 to prevent the water from electrically conducting the first region 211 or the second region 212 to the other regions. In some embodiments, the first region 211 and the second region 212 are formed at least by interweaving one or more insulating threads and a waterproof fiber to prevent the water from electrically conducting the first region 211 or the second region 212 to the other regions. More descriptions regarding embodiments of the various waterproof configuration described above may be found in the related descriptions below in the present disclosure.

In some embodiments, the first region 211 and the second region 212 are processed using one type of waterproof configuration. In some embodiments, the first region 211 and the second region 212 are processed using a plurality of types of waterproof configurations to prevent the water from electrically conducting the first region 211 or the second region 212 to the other regions in a greater extent. Merely by way of example, the waterproof structures 215 are disposed around the first region 211 and the second region 212, and the waterproof layers are also disposed on the first region 211 and the second region 212, thereby providing the plurality of types of the waterproof configurations for the first region 211 and the second region 212.

Some embodiments described later in the present disclosure will illustrate various implementation manners of the waterproof configuration.

In some embodiments, the waterproof structures 215 are respectively disposed around the first region 211 and the second region 212. A region around the first region 211 refers to a region on the inner surface of the garment 210 that surrounds the first region 211. The region around the first region 211 may "isolate" the first region 211 from the other regions of the inner surface. A region around the second region 212 refers to a region on the outer surface of the garment 210 that surrounds the second region 212. The region around the second region 212 may "isolate" the second region 212 from the other regions of the outer surface.

The waterproof structures 215 are configured to prevent moisture from penetrating into a peripheral region of the first region 211 and a peripheral region of the second region 212.

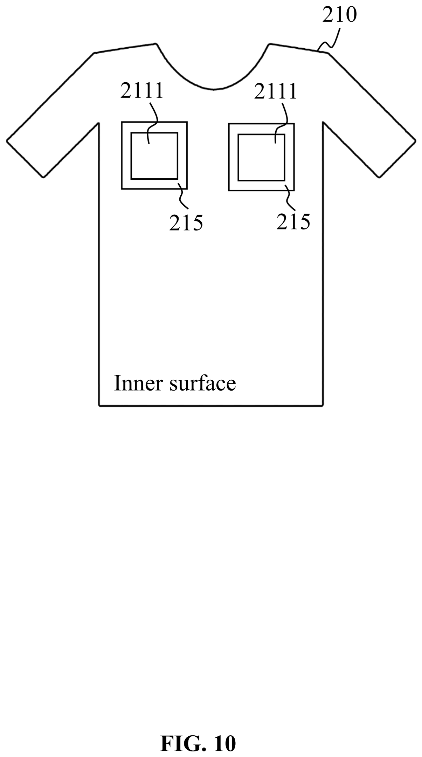

In some embodiments, the first region 211 is connected to the other regions of the garment 210 via at least one of the waterproof structures 215. The other regions refer to regions of the garment 210 except for the first region 211 and the second region 212. As shown in FIG. 10, the first region 211 is surrounded by at least one of the waterproof structures 215. The first region 211 is connected to the other regions of the garment 210 via the at least one of the waterproof structures 215. This configuration can prevent the first region 211 from being electrically connected to the other regions of the garment 210 through the water, reduce noise in the electrical signal acquired by the monitoring device, and improve the accuracy of the obtained electrical signal.

The waterproof configuration that the first region 211 is connected to the other regions of the garment 210 via at least one of the waterproof structures 215 is applicable to the various conductive electrode solutions described in the embodiments above in the present disclosure. For example, when the conductive electrode 220 is disposed via the one or more conductive threads 221, the waterproof structure 215 may "isolate" the first region 211 from the other regions, to prevent regions (e.g., the first region 211 and the second region 212) where the conductive electrode 220 is disposed from being electrically connected to the other regions of the garment 210. As another example, when the conductive electrode 220 is disposed via the conductive patches 222, the waterproof structure 215 may also "isolate" the first region 211 from the other regions, preventing the regions (e.g., the first region 211 and the second region 212) where the conductive patches 222 are disposed from being electrically connected to the other regions of the garment 210.

In some embodiments, the waterproof structures 215 may be obtained through a plurality of processing manners.

In some embodiments, at least one of the waterproof structures 215 is formed by weaving a waterproof fiber. The waterproof fiber may be made from polyurethane, thermoplastic polyurethane elastomer, etc. For example, during production of the garment 210, the peripheral region of the first region 211 is woven with the waterproof fiber to obtain the waterproof structure 215. Using the waterproof fiber to form the waterproof structure 215 can ensure the integrity of the garment 210 and improve comfort for the user when wears. As another example, after the initial weaving of the garment 210 is completed, the peripheral region of the first region 211 is subsequently woven through with the waterproof fiber to obtain the waterproof structure 215. As another example, during the production of the garment 210, the peripheral region of the first region 211 and the first region 211 are left un-woven. The first region 211 is produced separately. The peripheral region of the first region 211 is then woven with the waterproof fiber. Finally, the first region 211 and the peripheral region thereof are stitched to the garment 210.

In some embodiments, at least one of the waterproof structures 215 is formed by permeating a waterproof agent around the first region 211. The waterproof agent includes, but is not limited to, a fluorine-based waterproofing agent, a silicon-based waterproofing agent, etc. After the garment 210 is completed, the waterproof agent is permeated around the first region 211 to form the waterproof structure 215.

Using the waterproof agent to form the waterproof structure 215 simplifies the production process of the garment 210. Furthermore, the waterproof structure 215 of the garment 210 can be supplemented as needed to enhance the waterproof effect thereof. For example, when the waterproof effect of the original waterproof structure 215 of the garment 210 weakens due to wear or other reasons, a supplementary treatment with the waterproof agent may be applied to the original waterproof structure 215 to enhance the waterproof effect thereof.

The waterproof structure 215 formed by the waterproof fiber or the waterproof agent in the embodiments described above in the present disclosure is applicable to the various conductive electrode solutions described in the embodiments above in the present disclosure. For example, when the conductive electrode 220 is disposed using the one or more conductive threads 221, the waterproof structure 215 formed by the waterproof fiber or the waterproof agent does not affect the electrical connection between the first region 211 and the second region 212. As another example, when the conductive electrode 220 is disposed using the conductive patches 222, the waterproof structure 215 formed by the waterproof fiber or the waterproof agent also does not affect the electrical connection between the first region 211 and the second region 212.

In some embodiments, the second region 212 is also connected to other regions of the garment 210 via the waterproof structure 215.

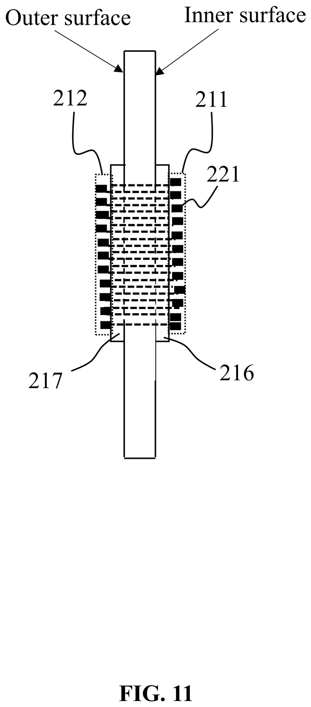

In some embodiments, as shown in FIG. 11, the garment 210 may further include a first waterproof layer 216 and a second waterproof layer 217. The first region 211 is disposed on a side of the first waterproof layer 216 facing the skin, and the second region 212 is disposed on a side of the second waterproof layer 217 facing away from the skin. The waterproof layer (e.g., the first waterproof layer 216 or the second waterproof layer 217) may be obtained by various manners. For example, the waterproof layer is formed by weaving a waterproof fiber or permeating a waterproof agent into a corresponding region. More descriptions regarding obtaining the waterproof layer using the waterproof fiber or the waterproof agent may be found in the related descriptions above in the present disclosure regarding obtaining the waterproof structure 215 using the waterproof fiber or the waterproof agent. As another example, during production of the garment 210, the region corresponding to the waterproof layer is left un-woven. After weaving other portions of the garment 210 is completed, a polyethylene film or another waterproof film is applied to the corresponding region of the garment 210 by various manners (e.g., stitching, gluing, etc.) to obtain the waterproof layer.

Since the first region 211 is disposed on the side of the first waterproof layer 216 facing the skin, the second region 212 is disposed on the side of the second waterproof layer 217 facing away from the skin, and the first waterproof layer 216 and the second waterproof layer 217 themselves are not wetted by water, even if peripheral regions of the first region 211 and the second region 212 contain the water, the water does not transfer to the first waterproof layer 216 and the second waterproof layer 217. This configuration can prevent the corresponding regions (e.g., the first region 211 and the second region 212) from being electrically connected to other regions through the water due to being wetted in a thickness direction of the garment 210.

It can be understood that since the first region 211 is disposed on the first waterproof layer 216, a size and a shape of the first waterproof layer 216 may be the same as those of the first region 211, or the size of the first waterproof layer 216 may be larger than the size of the first region 211. Similarly, a size and a shape of the second waterproof layer 217 may be the same as those of the second region 212, or the size of the second waterproof layer 217 may be larger than the size of the second region 212.

In some embodiments, the first region 211 is disposed on the side of the first waterproof layer 216 facing the skin, and the second region 212 is disposed on the side of the second waterproof layer 217 facing away from the skin, which facilitates the electrical connection between the first region 211 and the second region 212 by the conductive electrode 220, while also preventing a region where the conductive electrode 220 is disposed from being electrically connected to other regions of the garment 210 through the water. For example, the one or more conductive threads 211 shown in FIG. 11 may pass through the side of the first waterproof layer 216 facing the skin and the side of the second waterproof layer 217 facing away from the skin, which achieves the electrical connection between the first region 211 and the second region 212 while preventing the region where the one or more conductive threads 211 are disposed from being electrically connected to other regions of the garment 210 through the water. As another example, one conductive patch 222 is disposed on the side of the first waterproof layer 216 facing the skin, and the other conductive patch 222 is disposed on the side of the second waterproof layer 217 facing away from the skin. The two conductive patches 222 may at least partially contact each other to achieve the electrical connection between the first region 211 and the second region 212, while also preventing the two conductive patches 222 from being electrically connected to the other regions of the garment 210 through the water.

In some embodiments of the present disclosure, the first waterproof layer 216 and the second waterproof layer 217 are disposed to prevent the first region 211 and the second region 212 from being electrically connected to the other regions of the garment 210 through the water, reduce noise in the electrical signal acquired by the monitoring device, and improve the accuracy of the obtained electrical signal.

The basic concepts have been described above. Obviously, to those skilled in the art, the detailed disclosure above is merely an example and does not constitute a limitation on the present disclosure. Although not explicitly stated herein, a person skilled in the art may make various modifications, improvements, and corrections to the present disclosure. Such modifications, improvements, and corrections are suggested in the present disclosure, so they still fall within the spirit and scope of the exemplary embodiments of the present disclosure.

Meanwhile, the present disclosure uses specific words to describe the embodiments of the present disclosure. For example, "an embodiment," "one embodiment," and/or "some embodiments" mean a certain feature, structure, or characteristic related to at least one embodiment of the present disclosure. Therefore, it should be emphasized and noted that "an embodiment" or "one embodiment" or "an alternative embodiment" mentioned two or more times in different locations in the present disclosure does not necessarily refer to the same embodiment. In addition, certain features, structures, or characteristics in one or more embodiments of the present disclosure may be appropriately combined.

Furthermore, unless explicitly stated in the claims, the order of processing elements and sequences, the use of numbers and letters, or the use of other names in the present disclosure are not intended to limit the order of processes and manners of the present disclosure. Although the foregoing disclosure discusses some currently considered useful inventive embodiments through various examples, it should be understood that such details are for illustrative purposes only, and the appended claims are not limited to the disclosed embodiments. Instead, the claims are intended to cover all modifications and equivalent combinations that conform to the substance and scope of the embodiments of the present disclosure. For example, although the implementation of various components described above may be embodied in a hardware device, it may also be implemented as a software only solution, e.g., an installation on an existing server or mobile device.

Similarly, it should be noted that, in order to simplify the expression disclosed in the present disclosure and thereby facilitate the understanding of one or more inventive embodiments, in the foregoing description of the embodiments of the present disclosure, multiple features are sometimes grouped into one embodiment, drawing, or description thereof. However, this disclosure manner does not mean that the object of the present disclosure requires more features than those mentioned in the claims. Rather, claimed subject matter may lie in less than all features of a single foregoing disclosed embodiment.

In some embodiments, numbers describing the quantity of ingredients or attributes are used. It should be understood that such numbers used to describe the embodiments are modified by the modifiers "approximately," "about," or "substantially" in some examples. Unless otherwise stated, "approximately," "about," or "substantially" indicates that the stated number allows a variation of +20%. Accordingly, in some embodiments, the numerical parameters used in the specification and claims are approximate values, which may vary according to the characteristics required by individual embodiments. In some embodiments, numerical parameters should consider the specified number of significant digits and adopt the manner of general digit retention. Although the numerical ranges and parameters used to confirm the breadth of the scope in some embodiments of the present disclosure are approximate values, in specific embodiments, the disposing of such numerical values is as precise as possible within a feasible range.

For each patent, patent application, patent application publication, and other material, such as articles, books, specifications, publications, documents, etc., cited in the present disclosure, the entire content thereof is hereby incorporated by reference into the present disclosure. This excludes application history documents that are inconsistent with or conflict with the content of the present disclosure, and also excludes documents that limit the broadest scope of the claims of the present disclosure (whether currently or subsequently appended to the present disclosure). It should be noted that if the description, definition, and/or use of terms in the ancillary materials of the present disclosure are inconsistent with or conflict with the content described in the present disclosure, the description, definition, and/or use of terms in the present disclosure shall prevail.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A wearable device, comprising: a garment, including an inner surface configured to contact the skin of a user and an outer surface opposite to the skin, wherein:
the garment includes a conductive electrode configured to electrically connect a first region of the inner surface and a second region of the outer surface, the first region of the inner surface is configured to receive an electrical signal generated by the body of the user, and the second region of the outer surface is configured to connect to an external electrode such that the electrical signal is transmitted to the external electrode via the conductive electrode.

2. The wearable device according to claim 1, wherein the first region includes two first conductive regions, the two first conductive regions are disposed on a chest portion of the garment or are disposed on a waist portion of the garment; and
when the user wears the garment, the two first conductive regions are located on opposite sides of a midsagittal plane of the body of the user.

3. The wearable device according to claim 2, wherein the second region includes two second conductive regions, one of the two second conductive regions is electrically connected to one of the two first conductive regions, and the other of the two second conductive regions is electrically connected to the other of the two first conductive regions; and
for each second conductive region, a position of the second conductive region and a position of the first conductive region electrically connected to the second conductive region at least partially overlap along a circumferential direction and an axial direction of the body of the user.

4. The wearable device according to claim 2, wherein the second region includes two second conductive regions, one of the two second conductive regions is electrically connected to one of the two first conductive regions, and the other of the two second conductive regions is electrically connected to the other of the two first conductive regions; and
for each second conductive region, a position of the second conductive region and a position of the first conductive region electrically connected to the second conductive region do not overlap along a circumferential direction and an axial direction of the body of the user.

5. The wearable device according to claim 1, wherein the conductive electrode includes one or more conductive threads, a first portion of the one or more conductive threads is located on the outer surface, and a second portion of the one or more conductive threads extends to the inner surface and is electrically connected to the first portion of the one or more conductive threads.

6. The wearable device according to claim 5, wherein the first region and the second region are at least woven by one or more insulating threads, and the one or more conductive threads are interwoven through the inner surface and the outer surface.

7. The wearable device according to claim 6, wherein the second portion of the one or more conductive threads is woven to form a corrugated structure in the first region.

8. The wearable device according to claim 5, wherein the first region and the second region are formed by interweaving one or more insulating threads and the one or more conductive threads.

9. The wearable device according to any one of claims 5 to 8, wherein each of the one or more conductive threads is at least made by twisting a conductive fiber around an elastic fiber; or
the each of the one or more conductive threads is made by coating an outer side of the conductive fiber with an elastic film.

10. The wearable device according to claim 1, wherein the conductive electrode includes at least one set of conductive patches; and
for each set of conductive patches, one conductive patch of the set is disposed on the inner surface, another conductive patch of the set is disposed on the outer surface, and the conductive patch disposed on the inner surface and the conductive patch disposed on the outer surface are electrically connected by at least partially contacting each other.

11. The wearable device according to claim 10, wherein each conductive patch includes a conductive film or a metal electrode.

12. The wearable device according to claim 10, wherein each conductive patch includes a plurality of sub-conductive electrodes and wires electrically connecting the plurality of sub-conductive electrodes.

13. The wearable device according to claim 1, wherein the garment further includes an anti-slip region; and
the anti-slip region is located within the first region, or the anti-slip region is a peripheral region surrounding the first region.

14. The wearable device according to claim 13, wherein the anti-slip region is formed by weaving one or more anti-slip threads; or
the anti-slip region is coated with an anti-slip material.

15. The wearable device according to claim 1, wherein waterproof structures are respectively disposed around the first region and the second region.

16. The wearable device according to claim 15, wherein the first region is connected to other regions of the garment via at least one of the waterproof structures.

17. The wearable device according to claim 15, wherein at least one of the waterproof structures is formed by weaving a waterproof fiber; or
the at least one of the waterproof structures is formed by permeating a waterproof agent around the first region.

18. The wearable device according to claim 1, wherein the garment further includes a first waterproof layer and a second waterproof layer; and
the first region is disposed on a side of the first waterproof layer facing the skin, and the second region is disposed on a side of the second waterproof layer facing away from the skin.

19. The wearable device according to claim 1, wherein the second region includes two second conductive regions, and a corrugated structure is arranged between the two second conductive regions.
